# EUROPEAN PATENT SPECIFICATION

(11) **EP 1 004 329 B1**
(45) Date of publication and mention of the grant of the patent: **02.06.2004**
(21) Application number: 99203474.4
(22) Date of filing: 22.10.1999
(51) Int. Cl.: A61M 39/20

(54) **Connection hose for peritoneal dialysis with screw-sleeve terminal connector revolving around the hose**
Schlauchverbindung für Peritonealdialyse mit einer um den Schlauch drehbaren Schraubmuffe
Raccord pour tube utilisé en dialyse péritonéale, avec une bague filetée montée tournante autour du tube

(30) Priority: 25.11.1998 IT MI980763
(43) Date of publication of application: 31.05.2000
(73) Proprietor: HAEMOPHARM INDUSTRY AG, 9490 Vaduz (LI)
(72) Inventor: Valoti, Michele, 6900 Massagno (CH)
(74) Representative: Mittler, Enrico

(56) References cited:
- EP-A- 0 309 426
- EP-A- 0 462 355
- EP-A- 0 775 501
- WO-A-97/00095
- DE-A- 3 314 640

## Description

The present invention concerns a connection hose for peritoneal dialysis with terminal connector provided with a screw sleeve revolving around the hose.

A connection comprising the features defined in the preamble of claim 1 is disclosed in EP-A-0 775 501.

Among the peritoneal analysis techniques for the treatment of uraemic patients the Continuous Ambulatory Peritoneal Dialysis (CAPD) has a great importance.

This technique consists in connecting a container for dialysis liquid to a peritoneal catheter that is permanently associated with the patient. The connection is made by means of a pair of hoses, one permanently attached to the dialysis liquid container and the other one permanently attached to the peritoneal catheter, that are in turn couplable to each other by means of terminal connectors provided with screwable closing stoppers.

In order to carry out the coupling between the two hoses in condition of absolute sterility a device with communicating tanks filled with disinfecting liquid has been realized, in which respective threaded housings are made in which the closing stoppers of the terminal connectors of the aforesaid hoses are screwable each time, without any manual contact. This device is described in the international patent application WO-A-9700095.

Besides the several advantages the above described current technique has the inconvenience that the screwing operation involves the torsion of the hose to which the connector is attached with consequent repercussion at the abdominal wound in which the catheter is inserted and as much consequent suffering of the patient.

Object of the present invention is now to realise a connection hose for peritoneal dialysis with screwable terminal connector, that does not require the torsion of the hose for the screwing and unscrewing of the stopper in the housings of the sterile coupling device.

According to the invention such object is reached by means of a connection hose provided with a screwable end connector with removable stopper, as defined in claim 1.

Owing to the container thus made the screwing and unscrewing operation of the stopper does not involve the torsion of the hose but only the rotation of the external sleeve around the internal tubular body. Therefore it is possible to prevent any suffering to the patient

The characteristics of the present invention will be made more evident by the following detailed description of its embodiments that are illustrated as non limiting examples in the enclosed drawings, in which:

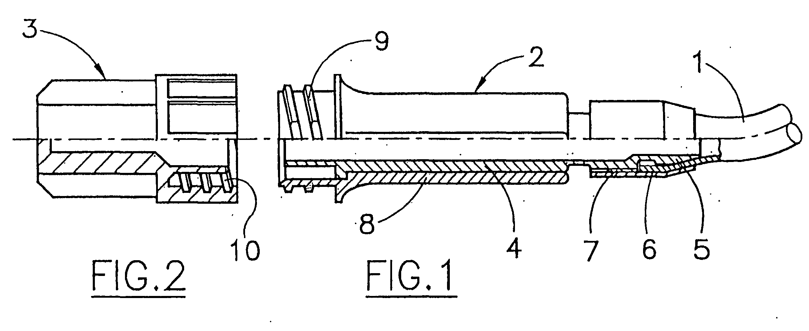

Figures 1 and 2 show respectively, half in longitudinal section and half in side view, a connection hose for peritoneal dialysis provided with a catheter side connector and the relative stopper;

In figure 1 a hose 1 for the catheter side connection is shown, that has a terminal connector 2 onto which a stopper like the one shown and indicated by 3 in figure 2 is screwable.

The connector 2 comprises a internal tubular body 4, that has a truncated-taper narrowing 5 on one end onto which the near end of the hose 1, that is kept in position and locked by a ring nut 6 screwed onto a male thread 7 of the inside tubular body 4 is inserted.

On the central part of the internal body 4 an external sleeve 8 is tumingly set, that in coincidence with the other end of the internal body 4 has a male thread 9 with which a female thread 10 of the stopper 3 is engageable.

Owing to the above described structure, with the stopper held in position in an opportune housing of the sterile connection device of the previously mentioned international patent application (or any other similar device for fixing the stopper) the connector 2 is separable from the stopper by simple rotation of the external sleeve 8, while the internal body 4 can remain still and not cause any torsions of the hose 1 that could be unpleasant for the patient under dialysis.

## Claims

1. Connection hose for peritoneal dialysis provided with a screw end connector (2) with removable stopper (3), said connector (2) is made up of a internal tubular body (4) fixable to the hose (1) and of an external sleeve (8) that is turningly mounted around said internal body (4) and provided with threading (9) for screw engagement with said stopper (3), **characterized in that** said internal tubular body (4) has a truncated-taper narrowing (5) at the other end onto which the near end of the hose (1) is inserted, that is kept in position and locked by a ring nut (6) that is screwed on a male thread (7) of the internal tubular body (4).

2. Connection hose according to the claim 1, **characterized in that** at one end of the internal tubular body (4) said sleeve (8) has a male thread (9) with which a female thread (10) of the stopper (3) is engageable.

## Patentansprüche

1. Schlauchverbindung für Peritonealdialyse mit
einem Schraubendverbinder (2) mit entfernbarem Stopfen (3),
wobei der Verbinder (2) aufgebaut ist aus:
einem inneren rohrförmigen Körper (4), der mit dem Schlauch (1) verbindbar ist, und
einer äußeren Hülse (8), die drehend um den inneren rohrförmigen Körper (4) herum angebracht und mit einem Gewinde (9) versehen ist zum Schraubeingriff mit dem Stopfen (3);
**dadurch gekennzeichnet, dass**
der innere rohrförmigen Körper (4) an dem anderen Ende eine abgeschnittene abgeschrägte Verjüngung (5) aufweist, auf die das nahe Ende des Schlauche (1) aufgesetzt wird, das von einer Ringmutter (6) in Position gehalten und verklemmt wird, die auf ein Außengewinde (7) des inneren rohrförmigen Körpers (4) geschraubt wird.

2. Schlauchverbindung nach Anspruch 1, **dadurch gekennzeichnet, dass** die Hülse (8) an einen Ende des inneren rohrförmigen Körpers (4) ein Außengewinde (9) aufweist, mit dem ein Innengewinde (10) des Stopfens (3) in Eingriff gebracht werden kann.

## Revendications

1. Tuyau souple de connexion pour dialyse péritonéale, équipé d'un connecteur terminal à vis (2) avec un arrêt amovible (3), ledit connecteur (2) étant constitué d'un corps tubulaire interne (4) susceptible d'être fixé sur le tuyau souple (1) et d'un fourreau externe (8) qui est monté en rotation autour dudit corps interne (4) et qui est pourvu d'un pas de vis (9) pour un engagement vissé avec ledit arrêt (3),
**caractérisé en ce que** ledit corps tubulaire interne (4) présente un rétrécissement en forme tronquée (5) à l'autre extrémité, sur lequel est insérée l'extrémité proximale du tuyau souple (1), qui est maintenue en en position et verrouillée par un écrou annulaire (6) qui est vissé sur un filetage (7) du corps tubulaire interne (4).

2. Tuyau souple de connexion selon la revendication 1, **caractérisé en ce que**, à une extrémité du corps tubulaire interne (4), ledit fourreau (8) comporte un filetage (9) avec lequel peut être engagé un taraudage (10) de l'arrêt (3).
